# EUROPEAN PATENT APPLICATION

(11) **EP 2 119 712 A1**
(43) Date of publication of application: **18.11.2009**
(21) Application number: 08703535.8
(22) Date of filing: 21.01.2008
(51) Int. Cl.: C07D 311/62, A23L 1/30, A61K 8/49, A61K 31/353, A61P 37/08, A61Q 1/00, A61Q 5/00, A61Q 19/00, C12P 17/06

(54) **NOVEL METHYLATED CATECHIN AND COMPOSITION CONTAINING THE SAME**

(30) Priority: 07.02.2007 JP 2007028419
(71) Applicant: Incorporated Administrative Agency National Agriculture and Food Research Organization, Tsukuba-shi Ibaraki 305-8517 (JP); ASAHI BREWERIES, Ltd., Sumida-ku Tokyo 130-8602 (JP)
(72) Inventor: YAMAMOTO, Mari, Kakegawa-shi Shizuoka 436-0069 (JP); KIRITA, Masanobu, Moriya-shi Ibaraki 302-0106 (JP); HONMA, Daiki, Moriya-shi Ibaraki 302-0106 (JP); YOKOTA, Toyokazu, Moriya-shi Ibaraki 302-0106 (JP)
(74) Representative: Vossius & Partner
(86) International application number: PCT/JP2008/050685
(87) International publication number: WO 2008/096586

(57) **Abstract**

A novel methylated catechin is provided which has stronger antiallergic activity than conventionally known methylated catechins such as epigallocatechin-3-O-(3-O-methyl)gallate and epigallocatechin-3-O-(4-O-methyl)gallate.

The epigallocatechin-3-O-gallate derivative is represented by the chemical formula I: 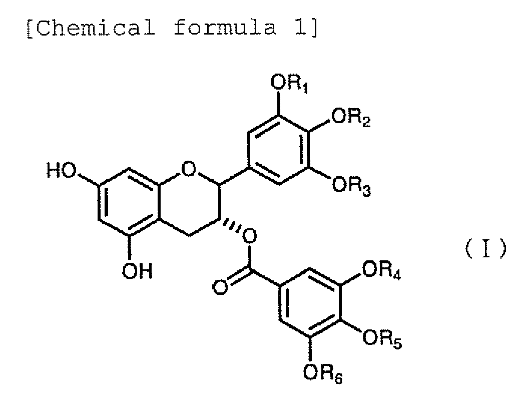 (wherein R₁ to R₆ are each a hydrogen atom or a methyl group, and at least three of R₁ to R₆ are methyl groups) or an isomer thereof.

## Description

### TECHNICAL FIELD

It is an object of the present invention to provide a novel methylated catechin produced by using, as a substrate, epigallocatechin-3-O-gallate, epicatechin-3-O-gallate, or an isomer thereof and to provide a method for manufacturing the same.

### BACKGROUND ART

In recent years, the number of allergic patients is rapidly increasing due to the changes in living environment. However, allergic diseases require long-term treatment. Therefore, there is a strong demand for allergy alleviating products that have no side effects and can be safely taken on a regular basis. Tea is a beverage favored by many people. Epigallocatechin-3-O-gallate (hereinafter, which may be abbreviated as "EGCG") is one of the main ingredients of tea and is known to have many physiological functions such as antiallergic activity (Non-Patent Document 1). Recently, catechins such as epigallocatechin-3-O-(3-O-methyl)gallate, and epigallocatechin-3-O-(4-O-methyl)gallate are found to have stronger antiallergic activity than EGCG (Patent Document 1 and Non-Patent Document 2).

Methods for methylating EGCG are described in Non-Patent Document 3 and Patent Documents 2 and 3. However, these methods are based on chemical synthesis and modification. Therefore, with these methods, it is very difficult to specifically methylate the hydroxy groups at positions 3, 4 and 5 of the galloyl group of EGCG. In another known method, a methylated catechin can be biosynthesized in an efficient and site-specific manner using a methylated catechin biosynthetic enzyme. However, this claimed method is for a compound prepared by methylating at least one hydroxy group of EGCG, and only methylation of up to two hydroxy groups is described in the Examples.
Patent Document 1: Japanese Patent Application Laid-Open No. 2000-159670.
Patent Document 2: Japanese Patent Application Laid-Open No. S61-145177.
Patent Document 3: Japanese Patent Application Laid-Open No. 2002-255810.
Non-Patent Document 1: Matsuo, N et al., Allergy 1997, 52, p58-64.
Non-Patent Document 2: Sano M, Suzuki M, Miyase T, Yoshino K, Maeda-Yamamoto M, J. Agric. Food Chem., 47(5), 1906-1910 (1999).
Non-Patent Document 3: J. Biochem. 55, p205 (1964).

### DISCLOSURE OF THE INVENTION

### PROBLEMS TO BE SOLVED BY THE INVENTION

The present invention provides a novel methylated catechin having stronger antiallergic activity than conventionally known methylated catechins such as epigallocatechin-3-O-(3-O-methyl)gallate and epigallocatechin-3-O-(4-O-methyl)gallate. The novel methylated catechin of the invention is produced using an enzyme reaction method in which EGCG derived from a natural product is used as a substrate. The invention also provides a method for manufacturing such a methylated catechin.

### MEANS FOR SOLVING THE PROBLEMS

The present inventors have isolated a methylated catechin synthetic enzyme gene according to the method described in Patent Document 4 (Japanese Patent Application Laid-Open No. 2006-141242). The inventors have obtained the methylated catechin synthetic enzyme by transforming the isolated gene into E. coli.

The present invention is described below.
(1) An epigallocatechin-3-O-gallate derivative represented by the chemical formula I:

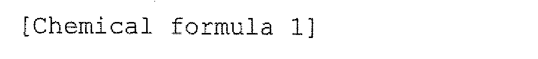

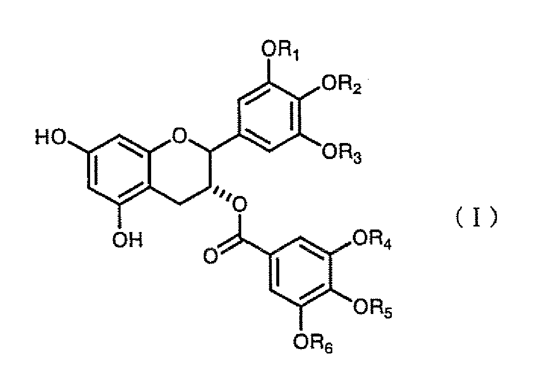

(wherein R₁ to R₆ are each a hydrogen atom or a methyl group, and at least three of R₁ to R₆ are methyl groups) or an isomer thereof.

(2) An epigallocatechin-3-O-gallate derivative represented by the chemical formula II:

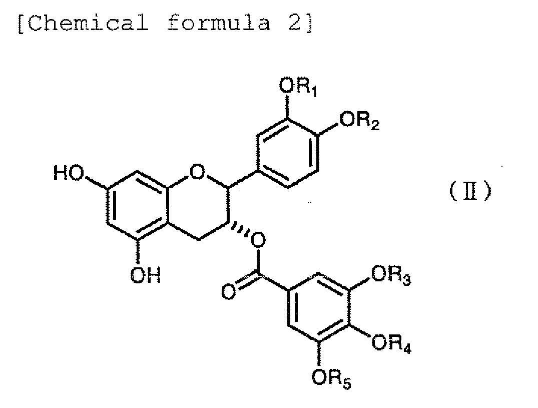

(wherein R₁ to R₅ are each a hydrogen atom or a methyl group, and at least three of R₁ to R₅ are methyl groups) or an isomer thereof.

(3) The epigallocatechin-3-O-qallate derivative of (1), wherein the epicatechin-3-O-gallate derivative is epi (3-O-methyl) gallocatechin-3-O- (3,5-O-dimethyl) gallate.

(4) A composition comprising a compound as set forth in any of (1) to (3).

(5) An antiallergic agent comprising a compound as set forth in any of (1) to (3).

(6) A food and beverage product comprising a compound as set forth in any of (1) to (3).

(7) A pharmaceutical or quasi drug comprising a compound as set forth in any of (1) to (3).

(8) A cosmetic comprising a compound as set forth in any of (1) to (3).

(9) A method for manufacturing a compound as set forth in any of (1) to (3), comprising reacting a methylated catechin biosynthetic enzyme with at least one substrate selected from the group consisting of epigallocatechin-3-O-gallate represented by the chemical formula III:

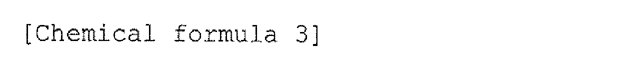

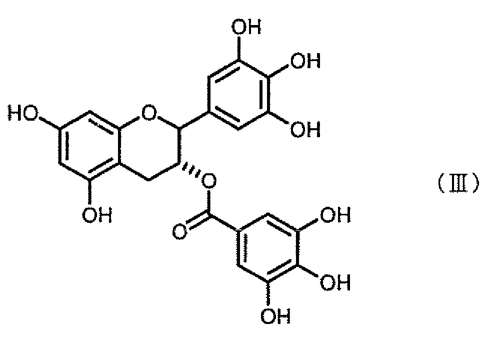

, isomers thereof, epicatechin-3-O-gallate represented by chemical formula IV:

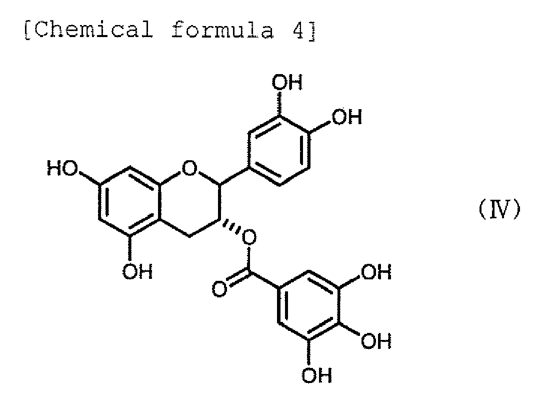

and isomers thereof.

### EFFECTS OF THE INVENTION

With the manufacturing method of the present invention, novel methylated catechins can be efficiently manufactured using catechins such as EGCG as substrates. The novel methylated catechins obtained by the invention have excellent effects such as antiallergic, anticancer, antiobesity, antiarteriosclerosis, antihypertensive, and antimicrobial effects and can be applied to various products such as food and beverage products, pharmaceutical drugs, quasi drugs, and cosmetics.

### BRIEF DESCRIPTION OF THE DRAWINGS

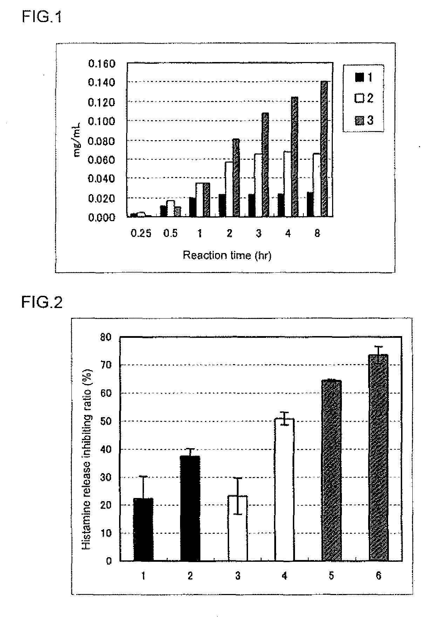

Fig. 1 is a graph showing the conversion efficiency of epigallocatechin-3-O-gallate to epi(3-O-methyl)gallocatechin-3-O-(3,5-O-dimethyl)gallate. In Fig. 1, numeral 1 denotes the addition of 10 mg of SAM to 10 mg of epigallocatechin-3-O-gallate; numeral 2 denotes the addition of 20 mg of SAM to 10 mg of epigallocatechin-3-O-gallate; and numeral 3 denotes the addition of 50 mg of SAM to 10 mg of epigallocatechin-3-O-gallate.
Fig. 2 is a graph showing the histamine release inhibitory activity in mouse mast cells. In Fig. 2, numeral 1 denotes 12.5 µg/mL of epigallocatechin-3-O-gallate; numeral 2 denotes 25 µg/mL of epigallocatechin-3-O-gallate; numeral 3 denotes 12.5 µg/mL of epigallocatechin-3-O-(3-O-methyl)gallate: numeral 4 denotes 25 µg/mL of epigallocatechin-3-O-(3-O-methyl)gallate; numeral 5 denotes 12.5 µg/mL of epi(3-O-methyl)gallocatechin-3-O-(3,5-O-dimethyl)gallate; and numeral 6 denotes 25 µg/mL of epi(3-O-methyl)gallocatechin-3-O-(3,5-O-dimethyl)gallate.

### BEST MODE FOR CARRYING OUT THE INVENTION

The novel methylated catechins of the present invention are compounds represented by the chemical formula I wherein at least three of R₁ to R₆ are methyl groups or compounds represented by the chemical formula II wherein at least three of R₁ to R₅ are methyl groups.

An enzyme for biosynthesizing methylated catechins (which may be referred to as "methylated catechin biosynthetic enzyme") is an enzyme that uses epigallocatechin-3-O-gallate, epicatechin-3-O-gallate, or an isomer thereof as a substrate to biosynthesize at least one methylated catechin selected from methylated catechins represented by the chemical formulas (I) and (II) and isomers thereof. Such an enzyme can be obtained by the method described in Japanese Patent Application Laid-Open No. 2006-141242.

An enzyme reaction mixture containing the novel methylated catechin of the invention can be obtained by suspending a methylated catechin biosynthetic enzyme protein in a buffer solution having a pH of 4.5 to 8.5 and preferably 6.5 to 8, adding EGCG and S-adenosyl-L-methionine (SAM) thereto, and allowing the mixture to react at 5 to 60°C and preferably 20 to 40°C.

A catechin compound containing the novel methylated catechin can be extracted by concentrating the obtained enzyme reaction mixture and using a solvent or the like that can extract the catechin compound. The novel methylated catechin can be manufactured by passing the catechin compound through a column filled with a synthetic adsorbent to adsorb the methylated catechin thereon, washing, and eluting with a suitable solvent. The novel methylated catechin of the invention can also be manufactured and obtained by subjecting the obtained catechin compound to preparative HPLC.

The compounds represented by the chemical formulas I and II and isomers thereof have antiallergic activity and are therefore useful as antiallergic agents.

The food and beverage product, pharmaceutical drug, quasi-drug, and cosmetic of the invention contain at least one compound selected from the group consisting of the compounds represented by the chemical formulas I and II and isomers thereof that are useful as antiallergic agents.

Example of the food and beverage product include: beverages such as soft drinks, carbonated drinks, energy drinks, fruit drinks, and fermented lactic drinks (including concentrated undiluted solutions thereof and powders for preparation of these drinks); frozen desserts such as ice creams, ice sherbets, and ice shavings; noodles such as buckwheat noodles, Japanese wheat noodles, gelatin noodles, dumpling skins, shaomai skins, Chinese noodles, and instant noodles; confectionery products such as candies, chewing gums, chocolates, tablets, snacks, biscuits, jellies, jams, creams, and baked goods; fishery and livestock processed foods such as fish pastes (kamaboko and chikuwa), hams, and sausages; milk products such as processed milk and fermented milk; fat and oil-based foods such as margarine, mayonnaise, shortening, whipped cream, and dressings; seasonings such as sauces and bastes; soups, stews, salads, side and main dishes, and pickles; and other various health foods, nutritional supplementary foods, and special health foods.

Examples of the pharmaceutical and quasi drugs include tablets, liquid medicines, capsules, drinkable preparations, and troches.

Examples of the cosmetic include: basic skin care products such as facial cleansing creams, skin lotions, packs, and skin care lotions; make-up cosmetics such as foundations, lipsticks, and eye shadows; body cosmetics such as nail enamels, soaps, bath additives, sunscreens, and spray deodorants; hair cosmetics such as shampoos, hair rinses, hair conditioners, and hair mousses; hair-skin cosmetics such as hair growth tonics, hair growth stimulants, and hair tonics; and aromatic cosmetics such as perfumes and colognes.

When these products are manufactured, the novel methylated catechin may be added together with generally used auxiliary raw materials and additives.

Examples of the auxiliary raw materials and additives include glucose, fructose, sucrose, maltose, sorbitol, stevioside, rubusoside, corn syrup, lactose, mannite, dextrin, citric acid, sodium citrate, tartaric acid, malic acid, succinic acid, lactic acid, L-ascorbic acid, dl-α-tocopherol, sodium erythorbate, glycerin, propylene glycol, glycerin fatty acid esters, sucrose fatty acid esters, sorbitan fatty acid esters, gum arabic, carrageenan, casein, gelatin, pectin, agar, vitamin C, vitamin Bs, vitamin E, nicotinic acid amide, calcium pantothenate, amino acids, calcium salts, surfactants, food colors, perfumes, and preservatives.

### Examples

Hereinafter, the present invention will be described in further detail by way of Examples. However, the scope of the invention is not limited to these Examples.

### [Example 1]

### (Isolation and purification of epi(3-O-methyl)gallocatechin-3-O-(3,5-O-dimethyl)gallate) (Enzyme reaction)

The enzyme reaction was performed using a crude enzyme solution of a methylated catechin biosynthetic enzyme manufactured by the method described in Japanese Patent Application Laid-Open No. 2006-141242. An enzyme reaction mixture (100 mM Tris-HCl (pH 7.4), 0.2 mM MgCl₂, 25 µM EGCG, 50 µM SAM, the amount of the crude enzyme solution was one-fifth of the amount of the enzyme reaction mixture) was prepared and allowed to react at 30°C for 16 hours. Subsequently, HCl was added to the reaction mixture such that the concentration of HCl in the reaction mixture was 0.04N to terminate the reaction. The reaction mixture was placed in a warm bath at 55°C for 30 minutes and then centrifuged (7,000 rpm x 10 minutes), and the supernatant was concentrated using a rotary evaporator. An equal amount of ethyl acetate was added to the concentrated solution, and the mixture was shaken and then centrifuged (7,000 rpm x 10 minutes) to collect the organic layer. The collected organic layer was concentrated and dried using a rotary evaporator and then dissolved in 35 (v/v)% methanol. The obtained 35 (v/v)% methanol solution was subjected to measurement using preparative HPLC to afford the product.

### Preparative HPLC conditions

Column: Inertsil ODS-3 (20 mm x 250 mm, GL Sciences Inc.) Inertsil ODS-3 guard column (20 mm x 50 mm, GL Sciences Inc.)
Mobile phase: 35 (v/v)% methanol
Flow rate: 12 mL/min, Detector: UV 280 nm
The peak fraction at about 40 min was collected under the above conditions, concentrated using a rotary evaporator, and freeze dried to give a powder of the title compound.

The molecular formula of the purified compound was determined by TOF-MS (time-of-flight mass spectrometry), and the structural formula was determined by NMR (nuclear magnetic resonance). TOF-MS measurement

### "UPLC/TOF-MS conditions"

HPLC: ACQUITY UPLC (Waters Corporation)
Column: ACQUITY UPLC BEH C18 (50 x 2.1 mm, 1.7 mm: Waters Corporation) Mobile phase: Solution A - methanol, solution B - 0.1 (v/v) % aqueous formic acid solution
   10 (v/v) % of solution A (0 min) → 50 (v/v)% of solution A (5 min)
Flow rate: 0.6 mL/min, column temperature: 40°C, injection amount: 5 mL
Detector: LCT Premier (Waters Corporation)
Ion source: ESI (positive, negative), cone voltage: 30 V, capillary voltage: 2500 V
Ion source temperature: 120°C, desolvation temperature: 300°C, MS scan: m/z 100-1000

A signal of [M+H]⁺ = 501 was observed in the positive ion mode measurement, and a signal of [M-H]⁻ = 499 was observed in the negative ion mode measurement. Therefore, the integer molecular weight was determined to be 500. From the results of the high resolution mass spectrometry, the molecular formula was determined to be C₂₅H₂₄O₁₁. NMR measurement
The purified compound was dissolved in CD₃OD. XWIN-NMR AV 600 (Bruker BioSpin) was used for the NMR measurement, and various NMR spectra (1H, 13C, HSQC, HMBC, and COSY) were measured. The measurement results are shown in Table 1. From the results, the compound was determined to be epi(3-O-methyl)gallocatechin-3-O-(3,5-O-dimethyl)gallate.

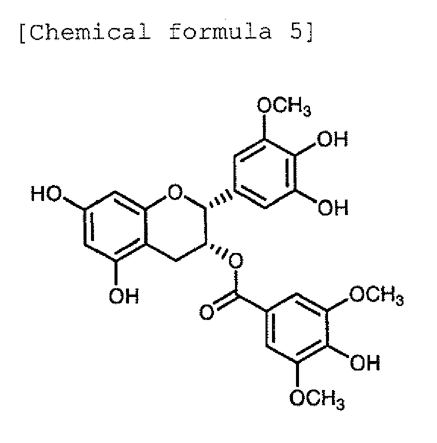

Epi(3-O-methyl)gallocatechin-3-O-(3,5-O-dimethyl)gallate

**[Table 1]**

| NMR measurement results of epi(3-O-methyl)gallocatechin-3-O-(3,5-O.dimethyl)gallate | | |
|---|---|---|
| | ¹³C-NMR δ (PPM) | ¹H-NMR δ (ppm), J value(Hz) |
| 2 | 78.8 | 5.07 (1H, br s) |
| 3 | 70.6 | 5.55 (1H, m) |
| 4 | 26.7 | 2.92 (1H, dd, J=3.0, 17.4) |
| | | 3.03 (1H, dd, J=4.8, 17.4) |
| 5 | 157.9^{a} | |
| 6 | 95.7 | 6,00 (1H d, J=2.4) |
| 7 | 158.1^{a} | |
| 8 | 96.6 | 5.98 (1H, d, J=2.4) |
| 9 | 157.3 | |
| 10 | 99.4 | |
| 1' | 130.7 | |
| 2' | 103.2 | 6.58. (1H, d, J=1.8) |
| 3' | 149.4 | |
| 4' | 134.9 | |
| 5' | 146.4 | |
| 6' | 108.7 | 6.65 (1H, d, J=1.8) |
| 3'-OMe | 56.5 | 3.62 (3H, s) |
| 1" | 121.5 | |
| 2", 6" | 108.3 | 7.17 (2H, s) |
| 3", 5" | 148.9 | |
| 4" | 142.2 | |
| 7" | 167.3 | |
| 3"-OMe, 5"-OMe | 56.8 | 3.81 (6H, s) |

| | | |
|---|---|---|
| ^{a}. Assignments may be interchanged | | |

### [Example 2]

### (Conversion efficiency from epigallocatechin-3-O-gallate to epi(3-O-methyl)gallocatechin-3-O-(3,5-O-dimethyl)gallate)

The conversion efficiency from epigallocatechin-3-O-gallate to epi(3-O-methyl)gallocatechin-3-O-(3,5-O-dimethyl)gallate was determined. An enzyme reaction mixture (100 mM Tris-HCl (pH 7.4), 0.2 mM MgCl₂, the amount of the crude enzyme solution was one-fifth of the amount of the enzyme reaction mixture) was prepared. In this case, 10 mg, 20 mg, or 50 mg of SAM and 10 mg of epigallocatechin-3-O-gallate were added to the mixture, and the resultant mixture was allowed to react at 30°C. 5 mL of the reaction mixture was collected 0.25, 0.5, 1, 2, 3, 4, and 8 hours after the start of the reaction, and then HCl was added such that its concentration in the collected reaction mixture was 0.04N to terminate the reaction. 8 mL of ethyl acetate was added to the collected mixture, and the resultant mixture was stirred and centrifuged (3000 pm x 5 min) to collect the organic layer. 200 µL of 1% ascorbic acid was added to the organic layer, and the resultant mixture was concentrated under suction. The amount of the produced epi(3-O-methyl)gallocatechin-3-O-(3,5-O-dimethyl)gallate was measured by HPLC. The results showed that the amount of the produced epi(3-O-methyl)gallocatechin-3-O-(3,5-O-dimethyl)gallate increased as the reaction time increased and that the conversion efficiency tended to increase as the amount of the added SAM increased. The changes in conversion efficiency are shown in Fig. 1.

### [Example 3]

### (The histamine release inhibitory activity of epi(3-O-methyl)gallocatechin-3-O-(3,5-O-dimethyl)gallate)

The effect of epi(3-O-methyl)gallocatechin-3-O-(3,5-O-dimethyl)gallate was determined based on the histamine release inhibitory activity in mouse mast cells. Epigallocatechin-3-O-gallate and epigallocatechin-3-O-(3-O-methyl)gallate known to have stronger antiallergic activity than epigallocatechin-3-O-gallate were used as comparison substances. BMMC (mouse bone marrow-derived mast cells) were used as the mouse mast cells and cultured in RPMI 1640 medium supplemented with 10% inactivated FBS (fetal bovine serum), 10% D₁₁ culture supernatant (as a source of IL-3), 5mM sodium glutamate, and 50 µM 2-mercaptoethanol. The cells (1 x 10⁷ cells/mL) were sensitized with anti-DNP-mouse IgE antibody overnight. On the next day, the resultant cells were suspended in Tyrode's solution and incubated with a test sample for 10 minutes. Then, DNP-HSA (antigen) was added thereto to induce degranulation (20 minutes), and the amount of histamine in the supernatant was measured by liquid chromatography. The antiallergic activity was evaluated based on the relative value with respect to the value for the control (distilled water). More specifically, the lower the relative value, the stronger the antiallergic activity. As shown in Fig. 2, the results showed that the histamine release inhibitory activity of epi(3-O-methyl)gallocatechin-3-O-(3,5-O-dimethyl)gallate was stronger than those of the comparison substances (epigallocatechin-3-O-gallate and epigallocatechin-3-O-(3-O-methyl)gallate.

The above results showed that a novel methylated catechin having strong antiallergic activity was obtained.

### INDUSTRIAL APPLICABILITY

The novel methylated catechins of the invention (methylated catechins of the chemical formula I wherein at least three of R₁ to R₆ are methylated and methylated catechins of the chemical formula II wherein at least three of R₁ to R₅ are methylated) can be efficiently manufactured using, as substrates, catechins such as EGCG with a methylated catechin biosynthetic enzyme. The manufactured novel methylated catechins are excellent in antiallergic activity, and therefore the present invention is quite useful.

## Claims

1. An epigallocatechin-3-O-gallate derivative represented by the chemical formula I: 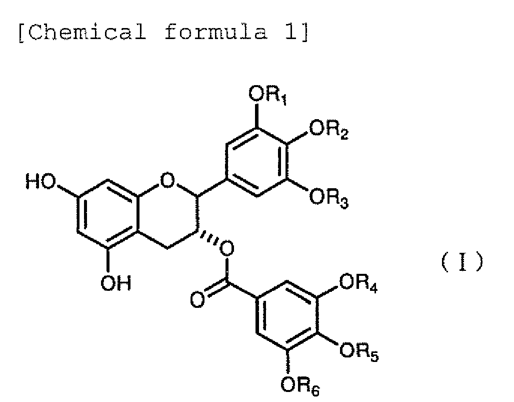 (wherein R₁ to R₆ are each a hydrogen atom or a methyl group, and at least three of R₁ to R₆ are methyl groups) or an isomer thereof.

2. An epicatechin-3-O-gallate derivative represented by the chemical formula II: 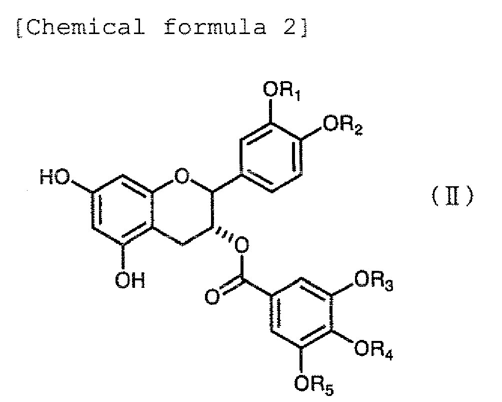 (wherein R₁ to R₅ are each a hydrogen atom or a methyl group, and at least three of R₁ to R₅ are methyl groups) or an isomer thereof.

3. The epigallocatechin-3-O-gallate derivative according to claim 1, wherein the epigallocatechin-3-O-gallate derivative is epi(3-O-methyl)gallocatechin-3-O-(3,5-O-dimethyl)gallate.

4. A composition comprising a compound as set forth in any of claims 1 to 3.

5. An antiallergic agent comprising a compound as set forth in any of claims 1 to 3.

6. A food and beverage product comprising a compound as set forth in any of claims 1 to 3.

7. A pharmaceutical or quasi drug comprising a compound as set forth in any of claims 1 to 3.

8. A cosmetic comprising a compound as set forth in any of claims 1 to 3.

9. A method for manufacturing a compound as set forth in any of claims 1 to 3, comprising reacting a methylated catechin biosynthetic enzyme with at least one substrate selected from the group consisting of epigallocatechin-3-O-gallate represented by the chemical formula III: 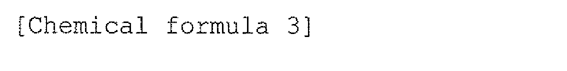 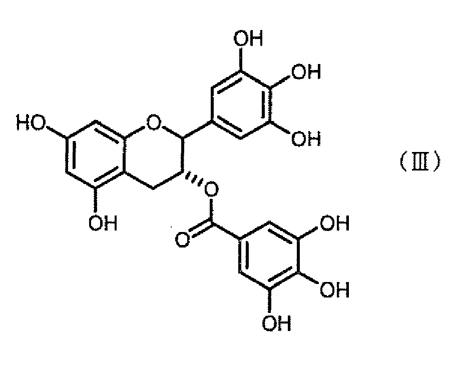 , isomers thereof, epicatechin-3-O-gallate represented by chemical formula IV: 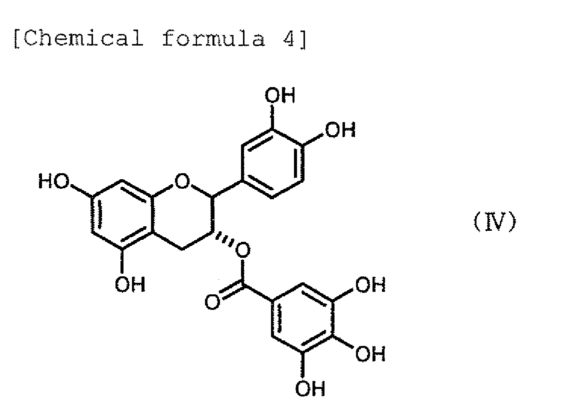 and isomers thereof.
